# FASCICULE DE BREVET EUROPEEN

(11) **EP 0 641 383 B1**
(45) Date de publication et mention de la délivrance du brevet: **13.03.2002**
(21) Numéro de dépôt: 93910109.3
(22) Date de dépôt: 11.05.1993
(51) Int. Cl.: C12N 5/04, C12N 15/82, A01H 4/00, A01H 5/00

(54) **CULTURE STABILISEE D'AGREGATS CELLULAIRES ET PROCEDE DE DEVELOPPEMENT DES EMBRYONS A PARTIR D'UNE SOUCHE PROEMBRYOGENE DESTINE A LA REGENERATION DE LA VIGNE**
STABILISIERTE KULTUR VON ZELLULARAGGREGATEN UND VERFAHREN ZUR ENTWICKLUNG VON EMBRYOS AUS PROEMBRYOGENEN STÄMMEN FÜR DIE REGENERATION VON WEINREBEN
CELLULAR AGGREGATE STABILIZED CULTURE AND PROCESS FOR THE DEVELOPMENT OF EMBRYOS FROM A PROEMBRYOGENIC STRAIN FOR USE IN VINE REGENERATION TECHNIQUES

(30) Priorité: 12.05.1992 FR 9205724
(43) Date de publication de la demande: 08.03.1995
(73) Titulaire: CHAMPAGNE MOET & CHANDON, 51200 Epernay (FR)
(72) Inventeur: MAURO, Marie-Claude, F-51270 Orbais-l'Abbaye (FR); DELOIRE, Alain, F-51100 Reims (FR); COUTOS-THEVENOT, Pierre, F-75005 Paris (FR)
(74) Mandataire: Warcoin, Jacques
(86) Numéro de dépôt international: FR9300456
(87) Numéro de publication internationale: WO9323529

(56) Documents cités:
- EP-A- 0 255 083
- WO-A-85/03085
- WO-A-91/05854
- DE-A- 3 126 001
- FR-A- 2 537 157
- US-A- 4 931 394
- JOURNAL AMERICAN SOCIETY OF HORTICULTURAL SCIENCE, vol. 113, no. 6, 1988, pages 941-945, St. Joseph, Michigan, US, J.A. STAMP et al.: "Proliferative Somatic Embryogenesis from Zygotic Embryos of Grapevine", le document en entier
- idem
- PLANT CELL REPORTS, vol. 7, no. 8, 1989, pages 684-687, Berlin, DE, N. MATSUTA et al. "Embryogenic cell lines from somatic embryos of grape (Vitis vinifera L.)", le document en entier
- HORTSIENCE, vol. 26, no. 6, page 772, St. Joseph, Michigan, US, D.J. GRAY et al.: "Perennial embryogenic cell cultures of grape - grapevine cell culture; application to somatic embryogenesis (conference abstract)", le document en entier
- BIOLOGICAL ABSTRACTS, vol. 81, 1980, abstract no. 161213, Philadelphia, PA, US, C. SRINIVISAN et al.: "High frequency somatic embryo production from unfertilized ovules of grapes" & SCI HORTIC 1980, vol. 13, no. 3, pages 245-252, abrégé

## Description

La présente invention concerne la régénération de la vigne par embryogénèse somatique et plus particulièrement une culture d'agrégats cellulaires proembryogènes et un procédé de développement des embryons à partir de ces cultures.

La régénération de la vigne, en tant que technique avale de la recherche, présente un intérêt essentiel dans l'amélioration de la vigne, ayant notamment un double objectif dans le domaine de la qualité des vins et de celui de la protection de l'environnement. Pour ce deuxième aspect en particulier, on comprend qu'il soit très important de pouvoir régénérer et multiplier, par exemple, des plants résistants à certaines maladies, afin de diminuer l'emploi de produits phytosanitaires souvent nuisibles pour l'environnement.

Dans le but d'améliorer les cépages, tout en préservant leurs caractéristiques organoleptiques des produits qui en découlent, différentes techniques de culture et de modification in vitro ont été testées notamment pour définir des cribles précoces de sélection, comme par exemple pour la résistance aux maladies (BRANCHARD M., Agronomie, 1984 4 (9), 905-911) ou pour la tolérance à la toxicité de certains éléments du sol (Bouquet A. et al., Coll. "Amélioration de la Vigne et Culture in vitro", Paris, 1985 147-157).

Afin de développer une telle technique de régénération de la vigne, il est toutefois nécessaire de disposer au moins des éléments suivants :
- une culture stabilisée de cellules proembryogènes pouvant être répliquées ou modifiées génétiquement ;
- un milieu de culture et une technique permettant sa réplication et son entretien ; et
- un milieu de culture et une technique favorisant la formation d'embryons et leur développement en. plantules.

Dans la présente description, on désignera par "plantule", une plante issue d'un embryon somatique, par analolgie à la plantule proprement dite issue normalement de la germination d'un embryon zygotique de la graine. Plus précisément encore, le Terme "plantule" utilisé dans la présente description, désigne la plante dans l'état où, après développement total de l'embryon, on distingue des cotylédons bien développés de couleur verte et une élongation racinaire.

Différents procédés de culture avaient été mis en oeuvre pour la réplication des cellules proembryogènes et le développement des embryons, notamment par la formation et le développement d'embryons secondaires tels que décrits dans US-A-4 532 733.

Toutefois, les techniques décrites rencontrent de nombreuses difficultés, tant au niveau de la formation des embryons qui sont généralement anormaux, qu'à celui du développement en plantules, celles-ci étant obtenues de ce fait avec des rendements particulièrement faibles.

En particulier, il a été observé que la formation d'embryons secondaires, souvent anormaux, conduisait en fait à diminuer le rendement en embryons développés, c'est-à-dire cotylédonaires, en fin de culture. Ainsi, ces difficultés ne permettaient pas d'envisager l'emploi de ces techniques en aval de résultats de recherche appliquée, en vue d'une production industrielle.

Par ailleurs, comme on le comprend aisément, les rendements en embryons développés et en plantules seront d'autant plus élevés, que les cellules de la culture utilisée présentent un important pouvoir embryogène.

La présente invention concerne donc en premier lieu une culture stabilisée d'agrégats cellulaires proembryogènes déposée le 29 avril 1992 sous le n° PL92042917 à la collection ECACC (European Collection of Animal Cell Cultures - Division of Biologies - Porton Down - Salisbury, Wiltshire, SP4 OJG, U.K.), leurs variants et les cultures dérivées, en particulier celles ayant conservé des propriétés proembryogènes équivalentes.

Par culture stabilisée, on entend une culture de cellules, ou d'agrégats cellulaires, dont la quantité d'ADN par noyau cellulaire reste identique au fil des réplications, et produisant au moyen de techniques de régénération une plante identique à la plante initiale, une telle stabilité pouvant s'étendre au-delà de 4 années de culture et de repiquages réguliers.

Les cellules sont principalement diploïdes à l'exception de celles d'entre elles en cours de mitose qui sont alors tétraploïdes.

Par variant ou culture dérivée, on entend les cellules modifiées, notamment par mutations, par réarrangements chromosomiques, par recombinaison génétique ou par phénomènes épigénétiques.

Par propriétés proembryogènes équivalentes, on entend une culture pouvant développer au moins 100 embryons par mg de cellules.

La culture déposée sous le n° PL92042917 ECACC est une souche de cellules embryogènes, se présentant sous la forme d'agrégats cellulaires, obtenue à partir d'anthères du porte greffe 41B, issu d'un croisement Vitis vinifera cv. Chasselasx Vitis berlandieri.

Pour cultiver et répliquer ces cellules, il est nécessaire de disposer d'un milieu de culture liquide et d'une technique appropriés afin d'empêcher la formation et le développement d'embryons, tout en permettant les mitoses.

Ainsi la présente invention concerne également un procédé pour entretenir une souche de cellules proembryogènes de vigne au moyen d'un milieu standard liquide de culture de cellules végétales, comme par exemple un milieu Murashige et Skoog (MS) ou Nitsch et Nitsch, supplémenté en auxine, à une concentration suffisante pour inhiber la différentiation cellulaire en embryons, tout en permettant les mitoses, et dont le pH est compris entre 5 et 6, ajusté par exemple avec une solution aqueuse de soude diluée.

Parmi les auxines, on peut citer l'acide 2,4-dichlorophénoxyacétique (2,4D), l'acide 1-naphtalèneacétique (NAA), l'acide 2-naphtoxyacétique (NOA).

Il a toutefois été observé que dans la mise en oeuvre du procédé de l'invention, l'effet des différentes auxines était plus ou moins bénéfique. Ainsi, l'auxine 2,4 D, bien que permettant une bonne multiplicati des cellules en début de culture, conduit souvent et assez rapidement, au bout de 2 ou 3 semaines à des nécroses, d'où une chute très sensible de la viabilité des cellules. L'action de l'ANA est différente : d'une part la multiplication des cellules est assez lente, et d'autre part un nombre assez important de cellules se différencient en embryons, ce qui est évidemment contraire au but recherché.

Par contre, il semble que le NOA procure les meilleurs résultats.

Ainsi, suivant un mode de réalisation avantageux du procédé de l'invention précité, on choisira comme auxine, l'acide 2-naphtoxyacétique (NOA).

Selon une variante de ce mode de réalisation, la concentration en NOA dans le milieu de culture est comprise entre 2,5 et 7,5 µM/litre et de préférence, elle est de 5 µM/litre environ.

Suivant encore un autre mode de réalisation du procédé précité, ladite souche constituée d'agrégats cellulaires est inoculée dans le milieu de culture à une densité comprise entre environ 3 et 8 mg de cellules fraiches par millilitre de milieu, puis on laisse croître la culture jusqu'à une densité de 3 à 5 fois supérieure, à la suite de quoi on revient par repiquage à une sous-culture de densité sensiblement identique à celle de l'inoculation initiale.

De préférence, préalablement à cette sous-culture, on effectue un tamisage des agrégats cellulaires afin de ne conserver pour réaliser la sous-culture que ceux dont la taille ne dépasse pas 500 µm.

En n'effectuant pas ce tamisage, on risquerait l'apparition de cellules différenciées en embryons, ce qui notamment par le fait que la différenciation se serait faite en présence d'auxine, conduiraient à un blocage de l'évolution de l'embryon à un stade précoce, et ce de manière irréversible.

Selon une variante préférée de ce mode de réalisation, on réalise la sous-culture précitée environ toute les trois semaines, tandis qu'on renouvelle le milieu de culture chaque semaine par repiquage.

Il apparaît qu'après quatre années en procédant comme indiqué ci-dessous, dans un tel milieu, la culture n° PL92042917 ECACC s'est développée tout en conservant son pouvoir embryogène et sa stabilité génétique.

On a ainsi constaté que la culture stabilisée précitée de la présente invention permettait de développer potentiellement 400 embryons par mg de cellules alors que l'art antérieur décrivait un potentiel embryogène d'un embryon par mg (LEBRUN L. et BRANCHARD M., 3ème symposium sur la physiologie de la vigne, Bordeaux, 1989, 38-41).

Un autre aspect de la présente invention concerne un procédé permettant l'expression de ce pouvoir embryogène de manière à obtenir des plantules avec des rendements compatibles avec une exploitation industrielle, étant entendu que ces plantules ne peuvent être obtenues qu'à la condition que les embryons puissent se développer complètement sans donner naissance à des anomalies, comme pour les procédés antérieurs.

Or, il a été trouvé d'une manière inattendue, que le renouvellement du milieu de culture à intervalles réguliers jusqu'à l'obtention des plantules permettait d'obtenir un tel résultat.

La présente invention concerne donc également un procédé de développement des embryons à partir d'une souche proembryogène destiné à la régénération de la vigne, dans lequel la souche est cultivée dans un milieu de culture liquide approprié renouvellé à intervalles réguliers jusqu'à l'obtention de plantules sur lesquels on distingue deux cotylédons bien développés généralement de couleur verte, et une racine allongée.

On effectue avantageusement le renouvellement du milieu de culture par repiquage.

Avantageusement, à chaque renouvellement du milieu ou repiquage, on ramène la densité de la culture à la densité initiale d'inoculation.

D'une manière générale et en fonction de la densité de la culture lors de l'inoculation, la souche est cultivée entre 3 et 7 jours puis repiquée quotidiennement jusqu'à l'obtention de plantules.

La densité de la culture lors de l'inoculation est avantageusement comprise entre 0,5 et 2 µl de cellules sédimentées (1xg) par ml de milieu, de préférence entre 1 et 2 µl/ml. Ainsi, pour une densité comprise entre 1 et 2 µl, on débutera les renouvellements de milieu ou repiquages réguliers entre 3 et 4 jours après le début de la culture, alors qu'on les effectuerait entre le 5ème et le 6ème jour pour des densités inférieures.

Par milieu de culture liquide approprié, on entend un milieu standard liquide de culture de cellules végétales exempt d'auxine.

De préférence, il s'agit d'un milieu standard liquide, tel que le milieu Murashige et Skoog (1962) bien connu de l'homme du métier, exempt d'auxine et modifié de telle sorte qu'il ne contient qu'une concentration moitié en macroéléments, que le sucrose a été remplacé par du glycérol et du maltose et auquel a été ajouté un hydrolysat de caséine.

Dans le procédé selon l'invention, la souche est avantageusement constituée, lors de l'inoculation initiale pour l'initiation des embryons, d'agrégats cellulaires d'une taille comprise entre 200 et 500 µm.

De tels agrégats sont obtenus par culture de la souche, de préférence suivant le procédé d'entretien de la souche précédemment décrit en présence d'auxine, puis filtrés successivement par un filtre de porosité d'environ 500 µm pour éliminer les agrégats de taille supérieure, puis par un filtre de porosité d'environ 200 µm, pour ne conserver que les agrégats cellulaires compris entre 200 µm et 500 µm. Enfin, ces agrégats sont lavés avec de préférence le milieu de culture approprié précité, de manière à éliminer sensiblement toute trace d'auxine.

En opérant selon le procédé de l'invention, les embryons formés à partir des agrégats cellulaires passent successivement par les stades de développements suivants : globulaire, coeur, torpille et embryons matures ou plantules.

Il peut être avantageux, bien que non indispensable, d'ajouter au milieu de culture une cytokinine, telle que de la zéatine, à partir du moment où les embryons ont atteint le stade "torpille", afin de favoriser le développement ultérieur. Toutefois, la durée de la culture en présence de cytokinine ne doit pas dépasser la durée au-delà de laquelle pourrait apparaître des perturbations de la culture.

Cette durée, par exemple pour la zéatine est d'environ 5 jours. Dès que les plantules sont obtenues, elles sont transférées sur milieu solide, par exemple d'agar, en présence d'un milieu approprié, afin d'obtenir des plantes développées.

Les exemples ci-après permettent d'illustrer un mode de réalisation préférentielle du procédé selon l'invention, appliquée à la culture déposée sous le n° PL92042917 ECACC, sans toutefois chercher à en limiter la portée.

### Exemple 1 : Entretien de la souche embryogène

La culture d'agrégats cellulaires obtenues à partir d'anthères du porte greffe 41B est entretenue par culture et repiquage régulier dans un milieu standard liquide de culture de cellules végétales disponible dans le commerce, telles qu'un milieu Murashige et Skoog (MS) supplémenté en acide 2-naphtoxyacétique (NOA).

Les cultures sont réalisées dans des fioles Erlenmeyer de 250 ml avec 80 ml de milieu, sous agitation à environ 110 t/mn à 21°C, de préférence dans l'obscurité.

La concentration en auxine est de 5 µM/l, et le pH est ajusté à 5,8 par de la soude 0,1 N avant autoclavage du milieu à 120°C pendant 20 minutes.

La souche est ensuite inoculée à une densité comprise entre 3 et 8 mg de cellules fraiches par ml de milieu et on laisse croître la culture jusqu'à une densité de 3 à 5 fois supérieure. On effectue alors un tamisage des agrégats cellulaires sous un écran de nylon ayant des pores d'une taille de 500 µm de manière à éliminer les agrégats d'une dimension supérieure. La culture est repiquée dans le milieu MS supplémenté, haque semaine.

L'opération de tamisage est renouvelée à intervalles réguliers, environ toutes les deux ou trois semaines, en fonction de la densité du milieu de culture.

### Exemple 2 : Initiation et développement des embryons

Le milieu de culture employé pour le développement d'embryons somatique est un milieu de culture liquide MS modifié, comprenant notamment 18 g/l de maltose, et 4,6 g/l de glycérol remplaçant le sucrose et 1 g/l d'hydrolysat de caséine.

Ce milieu exempt d'auxine est employé à un pH de 5,8 ajusté par l'addition de soude 0,1N.

Le milieu de culture est autoclavé 20 min à 120°C. Les cultures cellulaires indifférenciées, provenant de la culture cellulaire entretenue selon le protocole de l'exemple 1, sont filtrées successivement au travers d'écrans de nylon ayant des pores d'une taille de 500 µm, puis de 200 µm, de manière à ne conserver pour l'inoculation de la culture que des agrégats cellulaires de taille comprise entre ces deux valeurs.

Les agrégats cellulaires indifférenciés retenus sur le second filtre sont alors lavés 3 fois avec du milieu MS modifié, puis mis en suspension dans 30 ml de ce milieu.

La densité cellulaire exprimée en µl de volume cellulaire par ml de milieu est déterminée après sédimentation (1xg) des cellules dans un tube gradué conique.

On inocule alors 80 ml de milieu de culture MS modifié avec une suspention d'agrégats cellulaires à une densité de 1 µl/ml dans une fiole Erlenmeyer de 250 ml.

Après 4 jours de culture, de préférence dans l'obscurité, on effectue quotidiennement un repiquage dans le milieu MS modifié jusqu'à l'obtention de plantules pour une durée moyenne de 15 à 17 jours.

La durée totale du développement des embryons passant par les différents stades de développement globulaires, coeurs puis torpille avant de donner une plantule est d'environ 20 jours. A l'apparition du stade torpille, environ 10 à 12 jours après le début de la culture, on ajoute 5 mg/l de zéatine, hormone favorisant le développement des cotylédons et de l'apex pour une durée de 5 jours maximum.

Les plantules sont alors transférés dans un milieu agar solide (milieu MS modifié comprenant une concentration moitié en micro et macro éléments 20 g/l de sucrose, 7 g/l de bacto-agar Difco, à pH 5,8) sans aucune addition de régulateur de croissance.

Des plantes développées sont obtenues par culture des plantules à 25°C, sous une intensité lumineuse de 40 à 50µE.m⁻².s⁻¹ (tube fluorescent Mazda fluo A9TFRS40/BI) avec une photopériode de 16 heures, pendant environ 40 jours.

Ce procédé mis en oeuvre à multiple reprises avec des agrégats cellulaires embryogènes permet d'obtenir environ de 60% à 95% de plantules par rapport au nombre d'embryons initialement formés.

Des données comparatives entre différents procédés de développement des embryons avec ou sans repiquage sont présentées-dans le tableau 1.

**TABLEAU I**

| Stades de développement (%) | | | | | |
|---|---|---|---|---|---|
| Conditions de transferts (1) | globulaire (2) | coeur (3) | torpille (4) | plantule (5) | plant (6) |
| 20 jours sans repiquage | 40 | 60 | 0 | 0 | 0 |
| 4 jours sans repiquage + 16 repiquages quotidiens | 0 | 1 | 8 | 91 | 80 |
| 10 jours sans repiquage + 16 repiquages quotidiens | 17 | 68 | 15 | 0 | 0 |

Tous les essais ont été réalisés à composition de milieu liquide et de densité d'inoculation initiale identiques.

Le tableau I présente d'une part (colonnes 2 à 6) le nombre d'embryons selon leur stade de développement pour cent embryons en fin de culture en milieu liquide (20 jours ou 26 jours), et d'autres part (colonne 6) le nombre de plantes développées normales, après quarante jours de culture sur milieu solide d'agar, pour cent plantules tranférées, comme indiqué précédemment.

On remarque que, selon les procédés antérieurs, en l'absence de repiquage quotidien (1ère ligne de résultats) les embryons restent bloqués au stade "coeur", voire au stade "globuliare", tandis que, selon le procédé de l'invention (2ème ligne de résultats), avec un repiquage quotidien pendant 16 jours, à partir du 4ème jour de culture, 91% des embryons sont parvenus au stade "plantules", et sont susceptibles ensuite de produire des plantes développées normales. Par contre, on observe (3ème ligne de résultats) que si les repiquages quotidiens ne sont pas effectués suffisamment tôt, il se produit un blocage du développement des embryons au stade torpille, et qu'ainsi aucune plantule ne pourrait être obtenue.

Ainsi, grâce au procédé selon l'invention, pour cent embryons produits à partir de la souche d'agrégats cellulaires embryogènes, on peut obtenir environ 90 plantules en fin de culture en milieu liquide et environ 75 plantes développées normales après transfert sur milieu solide, ce qui rend ce procédé compatible avec exploitation industrielle.

Les plantes développées obtenues par le procédé décrit ci-dessus sont alors mises en terre, après leur adaptation nécessaire aux conditions extérieures de culture, afin d'obtenir une vigne régénérée.

La présente invention concerne donc également la vigne régénérée par des plantes issus de plantules obtenues par le procédé décrit précédemment.

Enfin, lorsque l'on dispose d'une culture stabilisée d'agrégats cellulaires à haut pouvoir embryogène, d'un milieu approprié pour son entretien et un procédé efficace de développement des embryons, l'ensemble peut être employé d'une manière avantageuse pour les application suivantes :
- sélection par criblage de plants résistants, par exemple en présence d'une toxine ;
- multiplication conforme de la vigne ;
- obtention et fusion de protoplastes ;
- transformation de la vigne par mutagénèse ; et
- transformation de la vigne par transformation génétique.

Ce dernier point est particulièrement intéressant dans la mesure où les exemples transformations génétiques connues sont limitées par le développement ultérieur des cellules transformées en embryons.

En effet, dans les techniques connues de tranformations génétique de la vigne, on observe pour bon nombre de cellules transformées un blocage dans la différenciation en embryons. Ainsi, partant d'une souche de pouvoir embryogène élevé, on peut compter en général sur une quantité suffisante d'embryons transformés.

La présente invention concerne donc également l'utilisation de la culture stabilisée déposée sous le n° PL92042917 ECACC, leurs variants et cultures dérivées, en particulier celles ayant conservé des propriétés proembryogènes équivalentes pour la transformation des cellules par un vecteur approprié, suivie de la régénération en plante.

Par vecteur approprié on entend tout moyen permettant le transfert, l'intégration dans le génome, l'expression et la réplication d'un gène à l'intérieur d'une cellule végétale.

Parmi les vecteurs connus, seront employés de préférence, les dérivés d'Agrobacterium tels que décrit par exemple dans le brevet européen n° 0 176 112.

Pour les mêmes raisons, la présente invention concerne également l'utilisation de la culture stabilisée déposée sous le n° PL92042917, et leurs variants et cultures dérivées en particulier celles ayant conservé des propriétés proembryogènes équivalentes pour l'obtention de protoplastes de la vigne.

On emploiera principalement les méthodes d'obtention de protoplastes décrites d'une manière générale (Cell and Tissue Culture in Forestry, Vol. 2, Edit. JM. BONGA, Don J. DURZAN, Martines NIJHOFF Publishers 1987, DORDRECHT, BOSTON, LANCASTER), ou plus particulièrement pour la vigne (BESSIS R. & al., journées sur l'amélioration de la vigne et culture in-vitro, organisées par MOET-HENNESSY S.A., PARIS, Avril 1985, p.195-196 ; LEBRUN L., journées sur l'amélioration de la vigne et culture in-vitro, organisées par MOET-HENNESSY S.A., PARIS, Avril 1985, p. 215).

## Revendications

1. Procédé de développement des embryons à partir d'une souche proembryogène destiné à la régénération de la vigne, **caractérisé en ce que** la souche est cultivée entre 3 et 7 jours dans un milieu de culture liquide exempt d'auxine puis repiquée quotidiennement jusqu'à l'obtention de plantules sur lesquelles on distingue deux cotylédons bien développés généralement de couleur verte et une racine allongée.

2. Procédé selon la revendication 1, **caractérisé en ce qu'**à chaque repiquage on ramène la densité de la culture à la densité initiale d'inoculation.

3. Procédé selon l'une des revendications 1 à 2, **caractérisé en ce que** la densité d'inoculation des cellules est comprise entre 0,5 et 2 µl/ml, de préférence entre 1 et 2 µl/ml/

4. Procédé selon l'une des revendications 1 à 3, **caractérisé en ce que** la souche est constituée, lors de l'inoculation initiale, d'agrégats cellulaires d'une taille comprise entre 200 et 500 µm.

5. Procédé selon la revendication 4, **caractérisé en ce que** les agrégats sont obtenus par culture de la souche, de préférence dans un milieu de culture liquide approprié en présence d'auxine, puis sont filtrés successivement par un filtre de porosité d'environ 500 µm pour éliminer les agrégats de taille supérieure, puis par un filtre de porosité d'environ 200 µm et enfin sont lavés avec de préférence le milieu de culture liquide approprié exempt d'auxine, de manière à éliminer toute trace d'auxine.

6. Procédé selon l'une des revendications 1 à 5, **caractérisé en ce que** la souche est une culture stabilisée d'agrégats cellulaires proembryogènes déposée sous le n° PL92042917 à la collection ECACC, leurs variants et les cultures dérivées ayant conservé des propriétés proembryogènes équivalentes.

7. Procédé selon la revendication 6, **caractérisé en ce que** la culture stabilisée peut développer au moins 100 embryons par mg de cellules.

## Patentansprüche

1. Verfahren zur Entwicklung von Embryonen ausgehend von einem proembryogenen Stamm, der zur Regeneration von Weinreben bestimmt ist, **dadurch gekennzeichnet, dass** der Stamm zwischen 3 und 7 Tagen in einem flüssigen Kulturmedium, das frei von Auxin ist, kultiviert wird, dann täglich verpflanzt wird bis zum Erhalt von Keimlingen, bei welchen man zwei gut entwickelte Kotyledonen mit im Allgemeinen grüner Farbe und eine längliche Wurzel unterscheidet.

2. Verfahren nach Anspruch 1, **dadurch gekennzeichnet, dass** man bei jedem Verpflanzen die Dichte der Kultur zu der anfänglichen Dichte der Inokulation zurückbringt.

3. Verfahren nach einem der Ansprüche 1 bis 2, **dadurch gekennzeichnet, dass** die Dichte der Inokulation der Zellen zwischen 0,5 und 2 µl/ml, vorzugsweise zwischen 1 und 2 µl/ml, eingeschlossen ist.

4. Verfahren nach einem der Ansprüche 1 bis 3, **dadurch gekennzeichnet, dass** der Stamm bei der anfänglichen Inokulation aus Zellaggregaten mit einer Größe von 200 bis 500 µm zusammengesetzt ist.

5. Verfahren nach Anspruch 4, **dadurch gekennzeichnet, dass** die Aggregate durch eine Kultur des Stammes, vorzugsweise in einem geeigneten flüssigen Kulturmedium, in Anwesenheit von Auxin erhalten werden, dann nacheinander durch ein Filter mit einer Porosität von etwa 500 µm, um die Aggregate mit größerer Größe abzutrennen, dann durch ein Filter mit einer Porosität von etwa 200 um filtriert werden, und schließlich vorzugsweise mit dem geeigneten flüssigen Kulturmedium, das frei von Auxin ist, gewaschen werden, um jegliche Spur von Auxin zu entfernen.

6. Verfahren nach einem der Ansprüche 1 bis 5, **dadurch gekennzeichnet, dass** es sich bei dem Stamm um eine stabilisierte Kultur von proembryogenen Zellaggregaten, hinterlegt unter der Nr. PL92042917 bei der Sammlung ECACC, deren Abwandlungen und abgeleitete Kulturen, bei denen äquivalente proembryogene Eigenschaften erhalten sind, handelt.

7. Verfahren nach Anspruch 6, **dadurch gekennzeichnet, dass** die stabilisierte Kultur mindestens 100 Embryonen pro mg Zellen entwickeln kann.

## Claims

1. Process for developing embryos from a proembryogenic strain intended for the regeneration of vine, **characterized in that** the strain is cultured between 3 and 7 days in an auxin-free liquid culture medium and then subcultured daily until plantlets are obtained in which two generally green well-developed cotyledons and a long root are distinguished.

2. Process according to Claim 1, **characterized in that**, on each subculturing, the density of the culture is brought back to the initial inoculation density.

3. Process according to either of Claims 1 and 2, **characterized in that** the cell inoculation density is between 0.5 and 2 µl/ml, preferably between 1 and 2 µl/ml.

4. Process according to one of Claims 1 to 3, **characterized in that** the strain consists, during the initial inoculation, of cellular aggregates between 200 and 500 µm in size.

5. Process according to Claim 4, **characterized in that** the aggregates are obtained by culturing the strain, preferably in an appropriate liquid culture medium in the presence of auxin, then they are filtered successively using a filter having a porosity of about 500 µm in order to remove the larger-size aggregates, then using a filter having a porosity of about 200 µm and finally washed preferably with the appropriate auxin-free liquid culture medium so as to remove all traces of auxin.

6. Process according to one of Claims 1 to 5, **characterized in that** the strain is a stabilized culture of proembryogenic cellular aggregates deposited under No. PL92042917 at the ECACC collection, their variants and derived cultures which have preserved comparable proembryogenic properties.

7. Process according to Claim 6, **characterized in that** the stabilized culture can develop at least 100 embryos per mg of cells.
